# EUROPÄISCHE PATENTANMELDUNG

(11) **EP 0 829 486 A2**
(43) Veröffentlichungstag der Anmeldung: **18.03.1998**
(21) Anmeldenummer: 97114245.0
(22) Anmeldetag: 18.08.1997
(51) Int. Cl.: C07H 17/08, C12P 19/62, C12N 1/20

(54) **Neue Polyenantibiotika, 3874 H1 bis H6, Verfahren zu ihrer Herstellung und Verwendung**

(30) Priorität: 19.08.1996 DE 19633310; 28.11.1996 DE 19649349
(71) Anmelder: HOECHST AKTIENGESELLSCHAFT, 65929 Frankfurt am Main (DE)
(72) Erfinder: Vértesy, Laszlo, Dr., 65817 Eppstein (DE); Kurz, Michael, Dr., 65719 Hofheim (DE); Wink, Joachim, Dr., 63322 Rödermark (DE); Markus, Astrid, Dr., 65835 Liederbach (DE); Stahl, Wilhelm, Dr., 65510 Idstein (DE)

(57) **Zusammenfassung**

Verbindungen der Formeln,
- 3874 H1,: Summenformel: C₅₈H₈₆N₂O₁₈, MG 1099,3 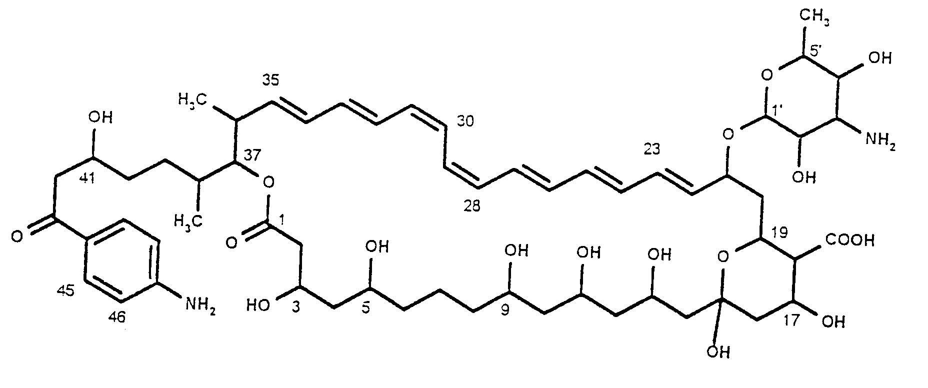
- 3874 H2,: Summenformel: C₅₉H₈₈N₂O₁₈, MG 1113,3 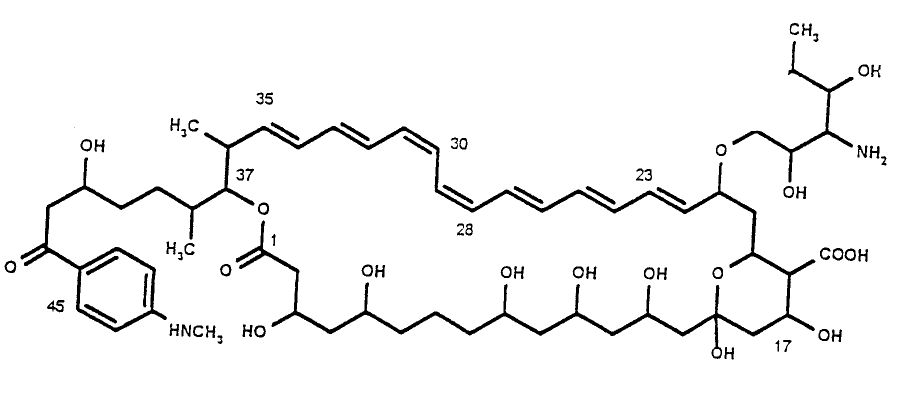
- 3874 H3,: Summenformel: C₅₇H₈₇NO₁₈, MG 1074,3 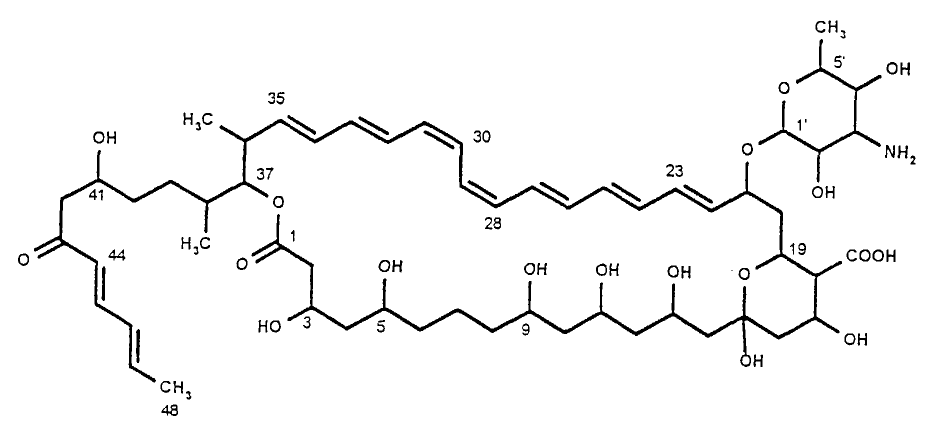
- 3874 H4,: Summenformel: C₅₈H₈₄N₂O₁₈, MG 1097,3
- 3874 H5,: Summenformel: C₅₉H₈₆N₂O₁₈, MG 1111,3
- 3874 H6,: Summenformel: C₅₇H₈₅NO₁₈, MG 1072,3.
eignen sich zur Behandlung von Pilzerkrankungen und zur Behandlung von Krankheiten, die mit einer erhöhten Konzentration von Steroiden einhergehen.

## Beschreibung

Die vorliegende Erfindung betrifft neue Heptaenantibiotika, Verfahren zu ihrer Herstellung und ihre Verwendung.

Es ist bereits eine große Anzahl von Heptaen-Antibiotika beschrieben worden. Heptaenantibiotika sind makrocyclische Lactone, die als charakteristisches Merkmal 7 miteinander konjugierte Doppelbindungen enthalten. Es sind mikrobiell gewonnene Naturstoffe, die als Antimycotika, als Mittel gegen Trichomonaden oder als Agenzien zur Bindung von Steroiden (Cholesterin) Verwendung finden. Es sind aber z. T. sehr toxische Verbindungen, die deswegen nicht systemisch (injiziert) werden, mit Ausnahme des Handelsproduktes Amphotericins B, (The Merck Index, eleventh edition, 1989, Seite 93) dem Urtyp der Polyenantibiotika. Wegen der zunehmenden Erkrankungen durch Pilze gibt es weiterhin einen großen Bedarf an neuen antifungischen Antibiotika, die entweder wirksamer oder besser verträglich sind, als die bekannten Polyen-Antibiotika.

Es wurde nun überraschend gefunden, daß die Streptomyces spec. HAG 3874, DSM 11007 neue, hoch aktive Heptaenantibiotika zu bilden vermag, die nicht nur sehr wirksam, sondern zum Teil auch gut verträglich sind.

Erfindungsgegenstand sind demzufolge die Verbindungen 3874 H1-H6 sowie ihre physiologisch verträglichen Salze sowie ihre offensichtlichen chemischen Äquivalente.
- 3874 H1,: Summenformel: C₅₈H₈₆N₂O₁₈, MG 1099,3 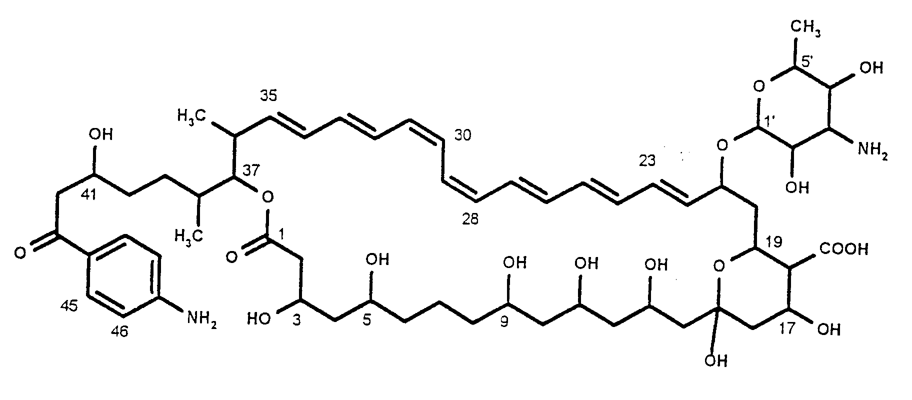
- 3874 H2,: Summenformel: C₅₉H₈₈N₂O₁₈, MG 1113,3 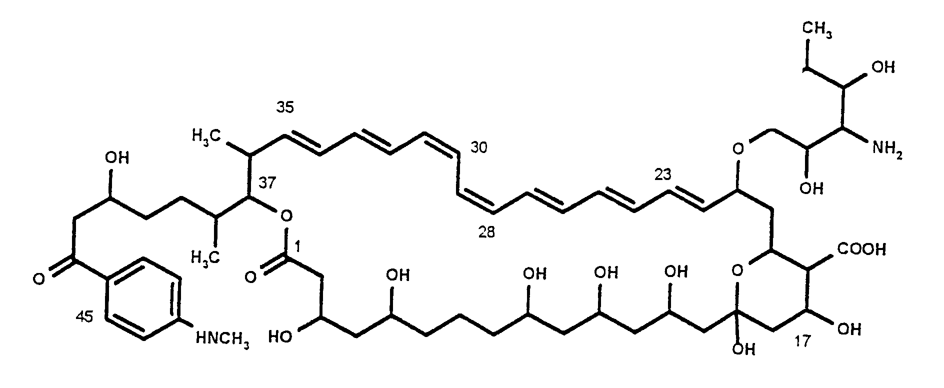
- 3874 H3,: Summenformel: C₅₇H₈₇NO₁₈, MG 1074,3 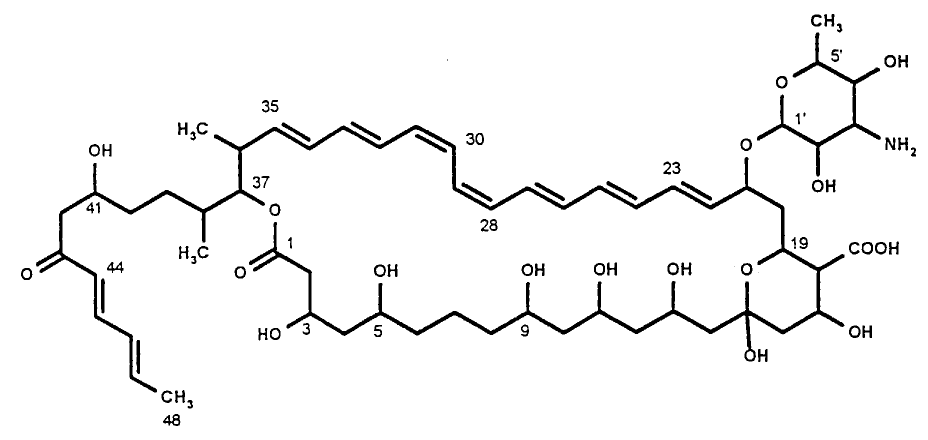
- 3874 H4,: Summenformel: C₅₈H₈₄N₂O₁₈, MG 1097,3
- 3874 H5,: Summenformel: C₅₉H₈₆N₂O₁₈, MG 1111,3
- 3874 H6,: Summenformel: C₅₇H₈₅NO₁₈, MG 1072,3.

Durch die angegebenen Struktur- bzw. Summenformeln und die beiden cis-Doppelbindungen in Δ 28/29 sowie Δ 30/31-Position, die in allen erfindungsgemäßen Verbindungen gleich sind, unterscheiden sich die Antibiotika 3874 H1 bis H6 von literaturbekannten Substanzen. Die erfindungsgemäßen Verbindungen weisen charakteristische Ultraviolett-Spektren auf.

Weiterhin gehören zum Gegenstand der vorliegenden Erfindung die Verfahren zur Herstellung der genannten Verbindungen. Ein Verfahren zur Herstellung der genannten Verbindungen ist dadurch gekennzeichnet, daß der Mikroorganismus Streptomyces species HAG 3874 (DSM 11007) in einem wäßrigen Nährmedium kultiviert wird und die Zielverbindungen anschließend isoliert und gereinigt werden. Der genannte Mikroorganismus ist am 21. Juni 1996 unter den Bedingungen des Budapester Vertrags bei der Deutschen Sammlung von Mikroorganismen und Zellkulturen, Mascheroder Weg 1b, D-38124 Braunschweig unter der Nummer DSM 11007 hinterlegt worden.

Streptomyces spec. DSM 11007 besitzt weißes Luftmycel und graue Sporenketten. Er bildet die für die Streptomyceten charakteristischen Sporenketten. In einer Nährlösung, die eine Kohlenstoffquelle und eine Stickstoffquelle sowie die üblichen anorganischen Salze enthält, produziert der Streptomyces spec. DSM 11007 die Verbindung 3874 H1 bis H6.

Anstelle des Stammes DSM 11007 können auch dessen Mutanten und Varianten eingesetzt werden, soweit sie die erfindungsgemäßen Verbindungen synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethylansulfonat (EMS); 2-Hydroxy-4-methoxy-benzophenon (MOB) oder N-Methyl-N'-nitro-N-nitrosoguanidin (MNNG) erzeugt werden.

Das Screening nach Mutanten und Varianten, die die erfindungsgemäßen Antibiotika produzieren, kann durch Bestimmung der biologischen Aktivität der in der Kulturbrühe angehäuften Wirkstoffe, beispielsweise durch Austesten der antimycotischen Wirkung nach dem unten beschriebenen Verfahren erfolgen.

Als bevorzugte Kohlenstoffquelle für die aerobe Fermentation eignen sich assimilierbare Kohlenhydrate und Zuckeralkohole, wie Glucose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Heptaene 3874 H1 bis H6 verläuft besonders gut z.B. in einer Nährlösung, die etwa 0.5 bis 5 % Glucose, vorzugsweise 1 bis 2 %, 0.5 bis 5 % Soyamehl, vorzugsweise 1 bis 2 %, Cornsteep, vorzugsweise 0.2 bis 1 %, 0.05 bis 1.0 % CaCO₃, vorzugsweise 0.1 bis 0.5 % und 0.1 bis 2 % NaCl, vorzugsweise 0.2 bis 1 %, jeweils bezogen auf das Gewicht der gesamten Nährlösung enthält.

Die Kultivierung erfolgt aerob, also beispielsweise submers unter Schütteln oder Rühren in Schüttelkolben oder Fermentern, gegebenenfalls unter Einführen von Luft oder Sauerstoff. Die Fermentation kann beispielsweise in Steilbrustflaschen oder Rundkolben verschiedener Volumina, in Glasfermentern oder V₂A-Stahltanks durchgeführt werden. Sie kann in einem Temperaturbereich von etwa 20 bis 35° C, vorzugsweise bei etwa 25 bis 30° C, durchgeführt werden. Der pH-Wert sollte zwischen 4 und 10 liegen, vorteilhaft zwischen 5,5 und 8,5. Man kultiviert den Mikroorganismus unter diesen Bedingungen im allgemeinen über einen Zeitraum von 20 bis 300 Stunden, bevorzugt 24 bis 140 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B. indem man ein versportes Mycel in eine Nährlösung überimpft und etwa 20 bis 120 Stunden, bevorzugt 24 bis 72 Stunden, wachsen läßt. Das versporte Mycel kann beispielsweise erhalten werden, indem man den Stamm etwa 1 bis 40 Tage, bevorzugt 3 bis 10 Tage, auf einem festen oder flüssigen Nährboden, beispielsweise Hefe-Malz-Agar oder Kartoffel-Dextrose-Agar, wachsen läßt.

Der Fermentationsverlauf und die Bildung der Antibiotika 3874 H1 bis H6 kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungsflüssigkeitschromatographie (HPLC) verfolgt werden.

Es ist eine Eigenart des Stammes HAG 3874 (DSM 11007), daß er neben den Heptaenen noch die literaturbekannten Antibiotika Carbazomycin B und Pimprinin zu bilden vermag.

Die Antibiotika 3874 H1 bis H6 können sowohl im Mycel als auch im Kulturfiltrat vorkommen, gewöhnlich befindet sich die Hauptmenge in der Zellmasse (Mycel). Es ist deshalb zweckmäßig, diese durch Filtration oder Zentrifugation vom Filtrat zu trennen. Das Filtrat wird mit einem mit Wasser nicht mischbaren Lösungsmittel wie z. B. 1-Butanol, Essigsäureethylester, Chloroform oder ähnlichem extrahiert. Das Mycel wird zweckmäßigerweise mit Methanol oder Aceton extrahiert, es können aber auch die oben genannten mit Wasser nicht mischbaren Lösungsmittel verwendet werden.

Die Extraktionen können in einem weiten pH-Bereich durchgeführt werden, es ist jedoch zweckmäßig, im neutralen Milieu, vorzugsweise zwischen pH 5 und pH 9 zu arbeiten. Die organischen Extrakte können z. B. im Vakuum konzentriert und getrocknet werden.

Eine Methode der Isolierung der Heptaene 3874 H1 bis H6 ist die Lösungsverteilung in an sich bekannter Weise.

Eine andere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Die Trennungen können in einem weiten pH Bereich durchgeführt werden. Bevorzugt ist der Bereich pH 1 bis pH 13; besonders bevorzugt ist der Bereich pH 2 bis pH 12. Geeignet sind darüber hinaus zahlreiche Reverse Phase-Träger, z. B. RP ₁₈, wie sie z. B. im Rahmen der Hochdruckflüssigkeits-Chromatographie (HPLC) allgemein bekannt geworden sind.

Eine weitere Reinigungsmöglichkeit für die erfindungsgemäßen Antibiotika besteht in der Verwendung von sog. Normal-Phasen-Chromatographie-Trägern, wie z. B. Kieselgel oder Al₂O₃ oder anderen in an sich bekannter Weise. Geeignet sind hierfür viele Lösungen und ihre Mischungen, wie z. B. Chloroform / Methanol-Gemische, denen basische Lösungsmittel wie z. B. Pyridin hinzugefügt worden sind.

Ein alternatives Isolierungsverfahren ist die Verwendung von Molekularsieben, wie z. B. Fractogel® TSK HW-40, Sephadex® LH-20 und andere, in an sich bekannter Weise. Es ist darüber hinaus auch möglich, aus angereichertem Material die Heptaene durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 7 bis 10 und Kationenaustauschern vorzugsweise im pH-Bereich von 3 bis 7. Besonders geeignet ist hierfür die Verwendung von Pufferlösungen, denen man Anteile von organischen Lösungsmitteln hinzugefügt hat.

Die Antibiotika 3874 H1 bis H6 oder abgeleitete chemische Derivate können nach dem Fachmann bekannten Methoden in die entsprechenden pharmakologisch verträglichen Salze übergeführt werden.

Offensichtliche chemische Äquivalente der erfindungsgemäßen Verbindungen sind Verbindungen die einen geringfügigen chemischen Unterschied aufweisen also die gleiche Wirksamkeit haben oder sich unter milden Bedingungen in die erfindungsgemäßen Verbindungen umwandeln. Zu den genannten Äquivalenten gehören z.B. Ester, Aminoderivate, Komplexe oder Addukte der bzw. mit den erfindungsgemäßen Verbindungen.

Unter pharmakologisch verträglichen Salze der erfindungsgemäßen Verbindungen versteht man sowohl anorganische als auch organische Salze, wie sie in Remington's Pharmaceutical Sciences (17. Auflage, Seite 1418 (1985)) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, H₂SO₄, Maleinsäure, Fumarsäure in Frage.

Die physikalisch-chemischen sowie spektroskopischen Eigenschaften der erfindungsgemäßen Antibiotika lassen sich wie folgt zusammenfassen:

### 3874 H1

Aussehen:
   grüngelbe, in Methanol, Acetonitril und Chloroform lösliche Substanz. Stabil in neutralem und mild alkalischem Milieu, jedoch unbeständig in saurer und stark-alkalischer Lösung sowie unter Licht-, Hitze- und Sauerstoffeinwirkung.
Summenformel: C₅₈H₈₆N₂O₁₈
Molekulargewicht: 1099,3
¹H-NMR: siehe Tabelle 1
UV-Maxima (log ε): 232 nm (4,49), 286 nm (4,32), 338 nm (4,64), 357 nm (4,86), 377 nm (5,00), 398 nm (4,98)

### 3874 H2

Aussehen:
   grüngelbe, in Methanol, Acetonitril und Chloroform lösliche Substanz. Stabil in neutralem und mild alkalischem Milieu, jedoch unbeständig in saurer und stark-alkalischer Lösung sowie unter Licht-, Wärme- und Sauerstoffeinwirkung.
Summenformel: C₅₉H₈₈N₂O₁₈
Molekulargewicht: 1113,3
UV-Maxima (log ε): 232 nm (4,49), 286 nm (4,32), 338 nm (4,64), 357 nm (4,86), 377 nm (5,00), 398 nm (4,98)

### 3874 H3

Aussehen:
   grüngelbe, in Methanol und anderen niederen Alkoholen, Acetonitril und Chloroform lösliche Substanz. Stabil in neutralem und mild alkalischem Milieu, jedoch unbeständig in saurer und stark-alkalischer Lösung sowie unter Licht-, Hitze- und Sauerstoffeinwirkung.
Summenformel: C₅₇H₈₇NO₁₃
Molekulargewicht: 1074,3
¹H-NMR: siehe Tabelle 1
UV-Maxima (log ε): 233 nm (4,39), 241 nm (4,39), 249 nm (4,28), 275 nm (4,41), 341 nm (4,54), 358 nm (4,82), 378 nm (5,00), 399 nm (4,94).

### 3874 H4

Aussehen:
   grüngelbe, in Methanol, Acetonitril und Chloroform lösliche Substanz. Stabil in neutralem und mild alkalischem Milieu, jedoch unbeständig in saurer und stark-alkalischer Lösung sowie unter Licht-, Hitze- und Sauerstoffeinwirkung.
Summenformel: C₅₈H₈₄N₂O₁₈
Molekulargewicht: 1097,3
UV-Maxima (log ε): 232 nm (4,49), 286 nm (4,32), 338 nm (4,64), 357 nm (4,86), 377 nm (5,00), 398 nm (4,98)

### 3874 H5

Aussehen:
   grüngelbe, in Methanol, Acetonitril und Chloroform lösliche Substanz. Stabil in neutralem und mild alkalischem Milieu, jedoch unbeständig in saurer und stark-alkalischer Lösung sowie unter Licht-, Hitze- und Sauerstoffeinwirkung.
Summenformel: C₅₉H₈₆N₂O₁₈
Molekulargewicht: 1111,3
UV-Maxima (log ε): 232 nm (4,49), 286 nm (4,32), 338 nm (4,64), 357 nm (4,86), 377 nm (5,00), 398 nm (4,98)

### 3874 H6

Aussehen:
   grüngelbe, in Methanol und anderen niederen Alkoholen, Acetonitril und Chloroform lösliche Substanz. Stabil in neutralem und mild alkalischem Milieu, jedoch unbeständig in saurer und stark-alkalischer Lösung sowie unter Licht-, Hitze- und Sauerstoffeinwirklung.
Summenformel: C₅₇H₈₅NO₁₃
Molekulargewicht: 1072,3
¹H-NMR: siehe Tabelle 1
UV-Maxima (log ε): 233 nm (4,39), 241 nm (4,39), 249 nm (4,28), 275 nm (4,41), 233 (4,39), 341 nm (4,54), 358 nm (4,82), 378 nm (5,00), 399 nm (4,94).

Durch die gute Löslichkeit unterscheiden sich die erfindungsgemäßen Antibiotika vorteilhaft vom Amphotericin B, welches in den genannten Lösungsmitteln sowie anderen nur sehr schwer löslich ist und dadurch bei der Anwendung große Probleme bereitet.

**Tabelle 1**

| Die chemischen Verschiebungen in den ¹H-NMR-Spektren von 3874 H3 und 3874 H1, aufgenommen in Deuteromethanol bei 17_{°} C. | | | | | |
|---|---|---|---|---|---|
| **Position am C-Atom** | **3874 H3** | **3874 H1** | **Position am C-Atom** | **3874 H3** | **3874 H1** |
| 1 | - | - | 29 | 6.61 | 6.61 |
| 2 | 2.49/2.14 | 2.45/2.15 | 30 | 6.51 | 6.51 |
| 3 | 4.28 | 4.28 | 31 | 6.06 | 6.06 |
| 4 | 1.66/1.41 | 1.66/1.41 | 32 | 6.82 | 6.82 |
| 5 | 3.36 | 3.37 | 33 | 6.22 | 6.20 |
| 6 | 1.30 | 1.30 | 34 | 6.19 | 6.20 |
| 7 | 1.84/1.12 | 1.85/1.11 | 35 | 5.38 | 5.38 |
| 8 | 1.31 | 1.30 | 36 | 2.48 | 2.48 |
| 9 | 3.69 | 3.70 | 36-Me | 1.02 | 1.02 |
| 10 | 1.44 | 1.43 | 37 | 4.76 | 4.77 |
| 11 | 4.04 | 4.05 | 38 | 1.85 | 1.86 |
| 12 | 1.52/1.25 | 1.51/1.38 | 38-Me | 0.98 | 0.98 |
| 13 | 4.43 | 4.42 | 39 | 1.36 | 1.40 |
| 14 | 1.72/1.56 | 1.72/1.55 | 40 | 1.58/1.46 | 1.62/1.51 |
| 15 | - | - | 41 | 3.97 | 4.06 |
| 16 | 2.00/1.24 | 1.99/1.24 | 42 | 2.68 | 2.98/2.92 |
| 17 | 4.25 | 4.25 | 43 | - | - |
| 18 | 1.99 | 1.99 | 44 | 6.10 | - |
| 18-CO | - | - | 45 | 7.23 | 7.75 |
| 19 | 4.38 | 4.39 | 46 | 6.26 | 6.62 |
| 20 | 2.24/1.68 | 2.24/1.67 | 47 | 6.28 | - |
| 21 | 4.37 | 4.38 | 48 | 1.87 | - |
| 22 | 6.10 | 6.10 | 1' | 4.53 | 4.54 |
| 23 | 6.17 | 6.17 | 2' | 3.85 | 3.88 |
| 24 | 6.50 | 6.49 | 3' | 2.71 | 2.80 |
| 25 | 6.35 | 6.35 | 4' | 3.12 | 3.17 |
| 26 | 6.50 | 6.49 | 5' | 3.21 | 3.23 |
| 27 | 6.79 | 6.79 | 5'-Me | 1.24 | 1.25 |
| 28 | 6.28 | 6.28 | | | |

Es wurde weiterhin gefunden, daß die erfindungsgemäßen Verbindungen außerordentlich starke fungizide, das heißt pilztötende Wirkungen aufweisen, darüber hinaus erstreckt sich die Aktivität auf ein breites Spektrum von Pilzgenera und Hefen. Tabelle 2 faßt die Minimalen Hemmkonzentrationen von 3874 H1 und 3874 H3 beispielhaft zusammen.

**Tabelle 2**

| In-Vitro Aktivität gegen Dermatophyten, Hefen und Schimmelpilze (Mikrodilutionstest in RPMI 1640 Medium) | | |
|---|---|---|
| STAMM | Minimale Hemmkonzentration, MHK (µg/ml) | |
| | 3874 H1 | 3874 H3 |
| Dermatophyten | | |
| T. mentagrophytes 100/25 | 4 | 8 |
| T. rubrum 101/58 | 16 | 16 |
| E. floccosum 190/143 | 16 | 16 |

| Hefen | | |
|---|---|---|
| C. albicans ATCC 90028 | 2 | 4 |
| C. albicans ATCC 90029 | 2 | 4 |
| C. glabrata ATCC 90030 | 4 | 4 |
| C. glabrata Berlin 12 | 2 | 4 |
| C. krusei 203/230 | 4 | 4 |
| C. krusei Berlin 1 | 2 | 2 |
| C. tropicalis 201/201 | 2 | 4 |
| C. tropicalis 201/202 | 2 | 4 |
| C. pseudotropicalis 202/218 | 2 | 4 |
| C. parapsilosis ATCC 90018 | 4 | 4 |
| C. neoformans ATCC90112 | 2 | 2 |

| Schimmelpilze | | |
|---|---|---|
| A. niger ATCC 16404 | 2 | 8 |
| A. fumigatus ATCC 9197 | 4 | 4 |
| A. flavus ATCC 9643 | 8 | 16 |
| Inkubation: 48 h bei 35°C (Hefen) oder 6 Tage bei 30°C (Haut- und Schimmelpilze) | | |

Die Überlegenheit der erfindungsgemäßen Antibiotika zeigt sich besonders in sogenannten Diffusionstesten, bei denen die Verbindungen in einer die Testkeime enthaltende Agarschicht diffundieren. Der Durchmesser der Hemmzonen ist dann ein Maß für die Wirksamkeit der Antibiotika (Tabelle 3).

**Tabelle 3**

| Konzentrationsabhängige Hemmzonen in mm, die von den Antibiotika 3874 H1 und H3 verursacht werden in Vergleich zu Amphotericin B | | | |
|---|---|---|---|
| Konzentration in mg/mL | 3874 H1 | 3874 H3 | Amphotericin B |
| 0.2 | 26 | 22 | 14 |
| 0.1 | 25 | 20 | 12 |
| 0.05 | 24 | 18 | 10 |
| 0.025 | 21 | 14 | 8 |
| 0.0125 | 19 | 13 | ø |
| 0.0063 | 17 | 12 | ø |

Die Wirkungen der erfindungsgemäßen Verbindungen übertreffen die des Handelsproduktes Amphotericins B z. T. erheblich und stellen daher sehr wertvolle Agenzien dar. Die Wirkung beruht vermutlich auf der Fähigkeit der neuen Heptaene Ergosterin als Heptaen/Ergosterin-Komplex zu binden. Ergosterin ist ein essentieller Bestandteil der pilzlichen Plasmamembranen. Durch die Komplexbildung wird in den Membranen das Ergosterin so verändert, daß die Struktur der Plasmamembran zerstört wird und die Pilzzelle stirbt.

Dem Ergosterin als Membranbestandteil entspricht in den Warmblüterzellen das Cholesterin. Viele literaturbekannte Heptaene binden das Cholesterin ebenso wie das Ergosterin und sind deshalb toxische Verbindungen. Durch die stärkere Bindung insbesondere der Antibiotika 3874 H3 und 3874 H6 an Ergosterin in Vergleich zu Cholesterin sind diese Verbindungen zur Bekämpfung von Pilzinfektionen in Menschen und Tieren besonders geeignet.

Neben der antimykotischen Wirkung besitzen die erfindungsgemäßen Antibiotika ausgezeichnete Hemmwirkungen gegen Protozoen, wie z. B. Trichomonas-Arten.

Aufgrund der Cholesterin- oder Sterol-Bindungsfähigkeit können aber die Verbindungen 3874 H1 bis H6 auch in der Medizin überall dort eingesetzt werden, wo zu hohe Konzentrationen von Steroiden unerwünscht sind. Beispiele sind hierfür die Verringerung von Cholesterinspiegel allgemein oder speziell die Behandlung von gutartigen Prostatahypertrophien.

Die vorliegende Erfindung betrifft demzufolge auch die Anwendung der erfindungsgemäßen Verbindungen als Arzneimittel, sowie die Verwendung der betreffenden Verbindungen zur Herstellung von Arzneimitteln zur Behandlung und/oder Prophylaxe von Pilzinfektionen, bzw. zur Behandlung von Erkrankungen, die mit einer erhöhten Konzentration von Steroiden einhergehen.

Des weiteren betrifft die vorliegende Erfindung Arzneimittel mit einem Gehalt an mindestens einer erfindungsgemäßen Verbindung.

Die erfindungsgemäßen Arzneimittel können enteral (oral), parenteral (intravenös), rektal oder lokal (topisch) angewendet werden. Sie können in Form von Lösungen, Pulvern, Tabletten, Kapseln (einschließlich Mikrokapseln), Salben (Cremes oder Gel), Liposomenpräparaten, Lipid-Komplexen, kolloidalen Dispersionen oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 10, vorzugsweise 0.2 - 8 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Durch die nachfolgenden Ausführungsbeispiele sowie durch den Inhalt der Patentansprüche soll die vorliegende Erfindung näher erläutert werden.

### Beispiel 1: Herstellung einer Sporensuspension des Produzentenstammes.

100 ml Nährlösung (20 g Malzextrakt, 2 g Hefeextrakt, 10 g Glucose, 0,5 g (NH₄)₂HPO₄ in 1 L Leitungswasser, pH-Wert vor der Sterilisation: 6,0) in einem 500 ml sterilen Erlenmeyerkolben werden mit dem Stamm beimpft und 72 Stunden bei 25°C und 140 UpM auf einer rotierenden Schüttelmaschine inkubiert. Anschließend werden 120 ml Kulturflüssigkeit in einem sterilen 500 ml Erlenmeyerkolben mit dem Nährboden Hafermehlinfus, 2,0 g/L, dem zusätzlich 15 g Agar/L zur Verfestigung zugegeben wurde, gleichmäßig verteilt und dekantiert. Die Kulturen werden 10 bis 14 Tage bei 25_{°} C inkubiert. Die nach dieser Zeit entstandenen Sporen eines Kolbens werden mit 500 ml entionisiertem Wasser, das einen Tropf eines handelsüblichen nichtionischen Tensids (z.B. ®Triton X 100, Fa. Serva) enthält, abgeschwemmt, sofort weiterverwendet oder bei -22° C in 50 % Glycerin oder in 10 % Dimethylsulfoxid bei -140° C aufbewahrt.

### Beispiel 2: Herstellung einer Kultur bzw. einer Vorkultur des Produzentenstammes im Erlenmeyerkolben.

Ein steriler 500 ml Erlenmeyerkolben mit 100 ml der im Beispiel 1 beschriebenen Nährlösung wird mit einer auf einem Schrägröhrchen gewachsenen Kultur oder mit 0,2 ml Sporensuspension angeimpft und auf einer Schüttelmaschine im Dunkeln bei 140 UpM und 25° C inkubiert. Die maximale Produktion der erfindungsgemäßen Verbindungen ist nach ca. 72 Stunden erreicht. Zum Animpfen von 10 und 100 L Fermentern genügt eine 72 Stunden alte Submerskultur (Animpfmenge ca. 5 %) aus der gleichen Nährlösung.

### Beispiel 3: Herstellung der Antibiotika 3874 H1- H6

Ein 10 L Fermenter wird unter folgenden Bedingungen betrieben:

| | | |
|---|---|---|
| Nährmedium: | Glukose | 15 g/L |
| | Sojamehl | 15 g/L |
| | Cornsteep | 5 g/L |
| | CaCO₃ | 2 g/L |
| | NaCl | 5 g/L |
| | pH 7.0 (vor dem sterilisieren) | |
| Inkubationszeit: | 24 oder 48 Stunden | |
| Inkubationstemperatur: | 25_{°} C | |
| Rührergeschwindigkeit: | 200 UpM, unter Lichtausschluß | |
| Belüftung: | 5 L Luft/min. | |

Durch wiederholte Zugabe weniger Tropfen ethanolischer Polyollösung kann die Schaumbildung unterdrückt werden. Das Produktionsmaxiumum wird nach 48 Stunden erreicht.

### Beispiel 4: Isolierung der Antibiotika 3874 H1 bis H6.

9 L der nach Beispiel 3 gewonnenen Kulturlösung werden abzentrifugiert und die Zellmasse (^{∼} 1,1 L) zweimal mit je 2,2 L Methanol ausgerührt. Die vereinigten Extrakte werden im Vakuum eingeengt, getrocknet und die Trockenmasse mit Diethylether digeriert. Der so gewaschene, entfettete Rückstand (41 g) wird in 25 % Isopropanol/75 % Wasser gelöst, auf eine 3 L fassende, mit dem Adsorptionsharz MCI Gel® CHP20P gefüllte Säule aufgetragen. Säulenmaße: Breite x Höhe: 11,3 cm x 30 cm. Eluiert wird mit einem Lösungsmittel-Gradienten von 25 % Isopropanol in Wasser bis 100 % Isopropanol und der Säulenausfluß in Fraktionen je 2 L aufgefangen.

Die heptaenhaltigen Fraktionen, die durch HPLC-Analysen überprüft werden, werden gesammelt und im Vakuum konzentriert sowie gefriergetrocknet (3,2 g).

### Beispiel 5: Hochdruckflüssigkeitschromatographie (HPLC) der Heptaene 3874 H1 bis H6.

- Säule:: Nucleosil® 100 - 5 C₁₈AB, 250/4.
- Mobile Phase:: 37,5 % Acetonitril in 10 mM Kaliumphosphat-Puffer, pH 7.0
- Flußgeschwindigkeit:: 1 mL pro Minute

Detektion durch UV-Absorption bei 320 nm.

Es werden für die einzelnen Komponenten folgende Retentionszeiten gefunden. Daneben sind die durch HPLC-Massenspektrometrie ermittelten, entsprechenden Molekulargewichte (M + H)⁺ angegeben. Für die HPLC-MS wird an Stelle von Phosphatpuffer 10 mM Ammonium-Acetat verwendet.

| Retentionszeit | Verbindung | (M + H)⁺ |
|---|---|---|
| 7,05 Min | 3874 H1 | 1099,6 |
| 8,64 Min | 3874 H4 | 1097,7 |
| 13,93 Min | 3874 H2 | 1113,7 |
| 17,90 Min | 3874 H5 | 1111,8 |
| 19,23 Min | 3874 H3 | 1074,5 |
| 24,28 Min | 3874 H6 | 1072,5 |

### Beispiel 6: Anreicherung der 3874 H-Komponenten.

2 g des nach Beispiel 4 gewonnenen Produktes werden auf eine 3 Liter fassende mit Fractogel® TSK MW-40 s gefüllte Säule (Breite x Höhe = 10 cm x 50 cm) aufgetragen. Das Laufmittel: Methanol wird mit einer Flußrate von 50 ml pro Minute über die Säule gepumpt und der Säulenausfluß fraktioniert (65 ml) aufgefangen. In den Fraktionen 28 bis 35 befindet sich hauptsächlich das Antibiotikum 3874 H3 (nach Trocknung: 210 mg), in den Fraktionen 39 - 43: H6 (9 mg), in den Fraktionen 50 - 60: 3874 H1 sowie H2 (280 mg) und schließlich in den Fraktionen 71 bis 78: die Verbindung 3874 H4 und H5 (17 mg).

### Beispiel 7: Endreinigung von 3874 H1, H2 und H3.

Die angereicherten Antibiotika 3874 H1 und H2 (280 mg) und 3874 H3 (210 mg), gewonnen nach Beispiel 6, werden jeweils auf einer Nucleosil® 12C₁₈AB-HPLC-Säule (Breite x Höhe = 3,2 cm x 25 cm) im Gradientenverfahren mit 25 % bis 50 % Acetonitril in Wasser aufgetrennt. Die durch analytische HPLC (siehe Beispiel 5) untersuchten Fraktionen werden entsprechend zusammengefaßt, im Vakuum eingeengt und gefriergetrocknet. Sie ergeben
29 mg 3874 H1 in 95 %iger Reinheit,
11 mg 3874 H2 in 94 %iger Reinheit,
65 mg 3874 H3 in 97 %iger Reinheit.

### Beispiel 8: Endreinigung durch präparative HPLC im Phosphatpuffer / Isopropanolsystem.

Verfahren wie in Beispiel 7, jedoch werden als Laufmittel 10 mM Kaliumphosphatpuffer, pH 7 sowie Isopropanol verwendet. Entsalzung der getrennten Komponenten wie im Beispiel 7.
38 mg 3874 H1 in 97 %iger Reinheit,
21 mg 3874 H2 in 96 %iger Reinheit,
83 mg 3874 H3 in 98 %iger Reinheit.

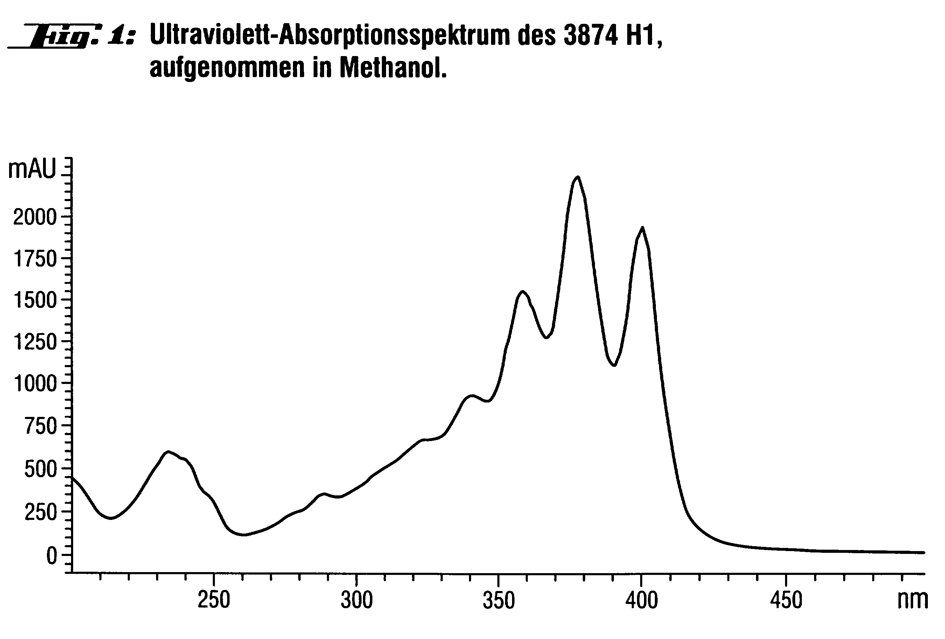

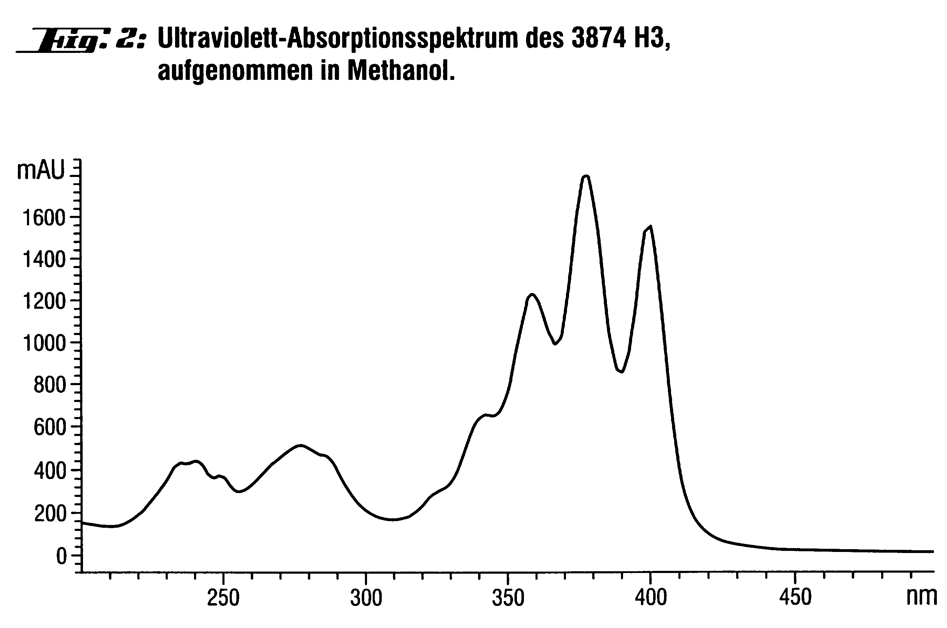

## Patentansprüche

1. Verbindungen 3874 H1 bis H6 mit den folgenden Formeln
3874 H1, Summenformel: C₅₈H₈₆N₂O₁₈, MG 1099,3 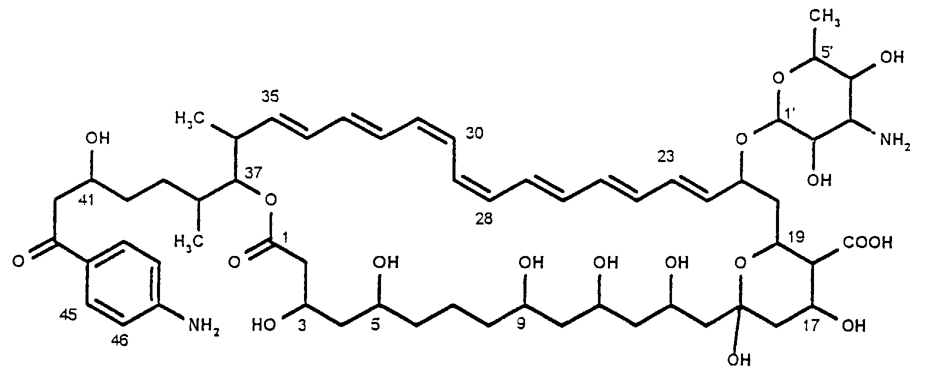
3874 H2, Summenformel: C₅₉H₈₈N₂O₁₈, MG 1113,3 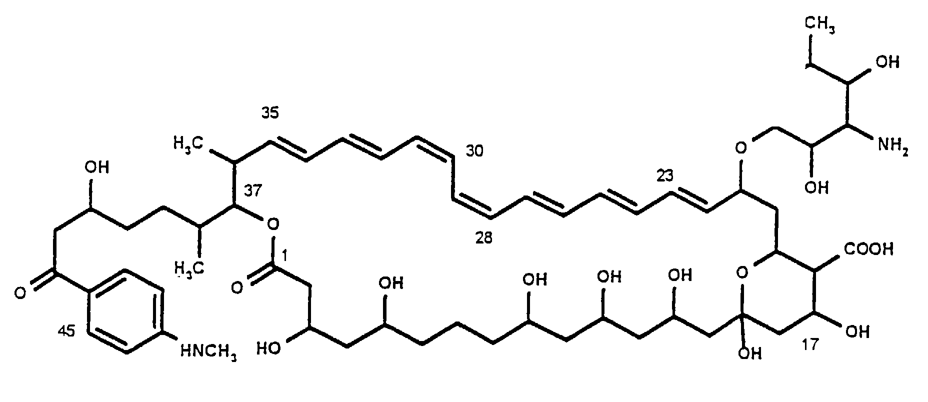
3874 H3, Summenformel: C₅₇H₈₇NO₁₈, MG 1074,3 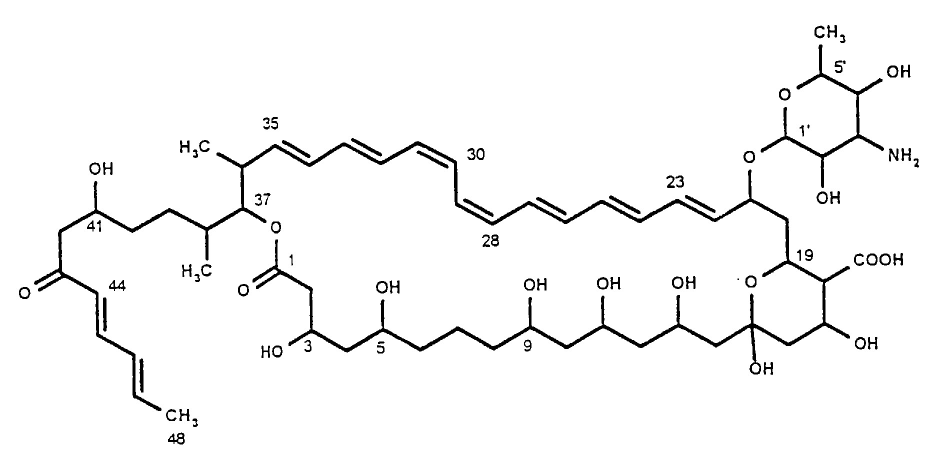
3874 H4, Summenformel: C₅₈H₈₄N₂O₁₈, MG 1097,3
3874 H5, Summenformel: C₅₉H₈₆N₂O₁₈, MG 1111,3
3874 H6, Summenformel: C₅₇H₈₅NO₁₈, MG 1072,3.
deren physiologisch verträgliche Salze sowie deren offensichtliche chemische Äquivalente.

2. Verbindungen gemäß Anspruch 1, herstellbar indem der Mikroorganismus DSM 11007 bzw. einer seiner Varianten oder Mutanten unter geeigneten Bedingungen fermentiert wird, eine oder mehrere der Verbindungen H1 bis H6 isoliert werden und ggf. in ihre Salze oder chemischen Äquivalente überführt werden.

3. Verfahren zur Herstellung von Verbindungen gemäß Anspruch 1, dadurch gekennzeichnet, daß der Mikroorganismus DSM 11007 bzw. einer seiner Varianten oder Mutanten unter geeigneten Bedingungen fermentiert wird, eine oder mehrere der Verbindungen H1 bis H6 isoliert werden und ggf. in ihre Salze oder chemischen Äquivalente überführt werden.

4. Verfahren gemäß Anspruch 3, dadurch gekennzeichnet, daß die Fermentation unter aeroben Bedingungen bei einer Temperatur zwischen 20 und 35 ^{°}C und bei einem pH zwischen 4 und 10 durchgeführt wird.

5. Verbindungen gemäß den Ansprüchen 1 oder 2 zur Anwendung als Arzneimittel.

6. Verwendung von Verbindungen gemaß den Anssprüchen 1 oder 2 zur Herstellung von Arzneimitteln zur Behandlung von Pilzerkrankungen oder Erkrankungen durch Trichomonaden.

7. Verwendung von Verbindungen gemäß den Ansprüchen 1 oder 2 zur Herstellung von Arzneimitteln zur Behandlung von Erkrankungen, die mit einer erhöhten Konzentration von Steroiden einhergehen.

8. Arzneimittel mit einem Gehalt an mindestens einer Verbindung gemäß den Ansprüchen 1 oder 2.

9. Verfahren zur Herstellung von Arzneimitteln gemäß Anspruch 8, dadurch gekennzeichnet, daß man mindestens eine Verbindung gemäß den Ansprüchen 1 oder 2 mit geeigneten Hilfs- und/oder Trägerstoffen in eine geeignete Darreichungsform bringt.

10. Streptomyces species DSM 11007.
